# EUROPEAN PATENT SPECIFICATION

(11) **EP 0 462 071 B1**
(45) Date of publication and mention of the grant of the patent: **01.02.1995**
(21) Application number: 91810450.6
(22) Date of filing: 13.06.1991
(51) Int. Cl.: A61K 38/04, A61K 47/28

(54) **Pharmaceutical resorption-improved somatostatin compositions, their preparation and use**
Pharmazeutische Somatostatin enthaltende Zusammensetzung mit verbesserter Resorption, seine Herstellung und Verwendung
Composition pharmaceutique de somatostatine à résorption améliorée, sa préparation et son utilisation

(30) Priority: 15.06.1990 GB 9013448
(43) Date of publication of application: 18.12.1991
(73) Proprietor: SANDOZ LTD., 4002 Basel (CH)
(72) Inventor: Fricker, Gerd, W-7813 Staufen (DE); Vonderscher, Jacky, F-68400 Riedisheim (FR)

(56) References cited:
- EP-A- 0 127 535
- WO-A-87/07149
- DERWENT FILE SUPPLIER WPIL, 1988, AN=88-45809 [07], Derwent Publications Ltd,London, GB; & JP-A-63 002 932
- GASTROENTEROLOGY, vol. 96, no. 5, part 2, 1989, page 580; L. BUSCAIL et al.:"Gallstone formation and occurrence of cholesterol monohydrate crystals in gallbladder bile of patients with long-term sandostatin treatment"

## Description

The invention relates to pharmaceutical compositions containing a somatostatin and a resorption promoter and having an improved somatostatin bioavailability, and to concomitant administration of a somatostatin and a resorption promoter.

The invention provides a pharmaceutical composition which contains a somatostatin and at least one cholanic acid of the formula I 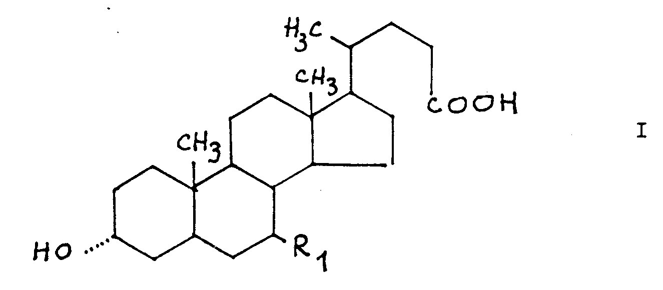
in which R₁ = α- or β-OH as a resorption promoter with a gallstone eliminating/preventing or size reducing activity, adapted for administration via a transmucosal route.

The naturally occuring somatostatin is one of the contemplated compounds and is a tetradecapeptide having the structure:- 
Ala-Gly-Cys-Lys-Asn-Phe-Phe-Trp
        *                 *
        Cys-Ser-Thr-Phe-Thr-Lys
This hormone is produced by the hypothalmus gland as well as other organs, e.g. the GI tract, and mediates, together with GRP, q.v. the neuroregulation of pituitary growth hormone release. In addition to inhibition of GH release by the pituitary, somatostatin is a potent inhibitor of a number of systems, including central and peripheral neural, gastrointestinal and vascular smooth muscle. It also inhibits the release of insulin and glucagon.

The term "somatostatin" includes its analogues or derivatives thereof. By derivatives and analogues is understood straight-chain, bridged or cyclic polypeptides wherein one or more amino acid units have been omitted and/or replaced by one or more other amino radical(s) of and/or wherein one or more functional groups have been replaced by one or more other functional groups and/or one or more groups have been replaced by one or several other isosteric groups. In general, the term covers all modified derivatives of a biologically active peptide which exhibit a qualitatively similar effect to that of the unmodified somatostatin peptide.

Agonist analogues of somatostatin are thus useful in replacing natural somatostatin in its effect on regulation of physiological functions.

Some of these offer improvements, e.g. in bioavailability and duration of action. However, little clinical evidence has yet been published on non-parenteral e.g. orally effective therapeutic somatostatin compositions.

Preferred known somatostatins are:-
a) 
        *                      *
(D)Phe-Cys-Phe-(D)Trp-Lys-Thr-Cys-Thr-ol
 (Generic name Octreotide)
b) 
        *                      *
(D)Phe-Cys-Tyr-(D)Trp-Lys-Val-Cys-ThrNH2
 (described in EP 203,031 A2; Generic name Vapreotide)
c) 
*                         *
(D)Phe-Cys-Tyr-(D)Trp-Lys-Val-Cys-TrpNH2
d) 
*                         *
(D)Trp-Cys-Phe-(D)Trp-Lys-Thr-Cys-ThrNH₂
e) 
        *                      *
(D)Phe-Cys-Phe-(D)Trp-Lys-Thr-Cys-ThrNH2
f) 
                        *                      *
3-(2-(Naphthyl)-(D)Ala-Cys-Tyr-(D)Trp-Lys-Val-Cys-ThrNH2
 (described in EP 214.872 A2 and EP 215.171 A2; Generic name Angiopeptin)
g) 
        *                      *
(D)Phe-Cys-Tyr-(D)Trp-Lys-Val-Cys-β-Nal-NH₂
h) 
                    *                      *
3-(2-Naphthyl)-Ala-Cys-Tyr-(D)Trp-Lys-Val-Cys-β-Nal-NH2
i) 
*                         *
(D)Phe-Cys-β-Nal-(D)Trp-Lys-Val-Cys-Thr-NH₂
 wherein in each of compounds a) to i) there is a bridge between the amino acids marked with a *.

Other contemplated somatostatins include:- 
*                                                    *
H-Cys-Phe-Phe-(D)Trp-Lys-Thr-Phe-Cys-OH
(See Vale et al., Metabolism, 27, Suppl. 1, 139 (1978)). 
*                                                    *
Asn-Phe-Phe-(D)Trp-Lys-Thr-Phe-Gaba
(See European Pat. Publication No. 1295) 
*                         *
MeAla-Tyr-(D)Trp-Lys-Val-Phe
(See Verber et al., Life Sciences, 34, 1371-1378 (1984), EP 0351.354 A2 and EP 70 021)) also known as cyclo (N-Me-Ala-Tyr-D-Trp-Lys-Val-Phe). 
*                         *
NMePhe-His-(D)Trp-Lys-Val-Ala
(See R.F.Nutt et al., Klin.Wochenschr. (1986) 64 (Suppl. VII) 
*                                                              *
H-Cys-His-His-Phe-Phe-(D)Trp-Lys-Thr-Phe-Thr-Ser-Cys-OH
(See EP-A-200,188)
wherein in the above mentioned amino acids there is a bridge between the amino acids marked with a *.

The contents of all the above publications including the specific compounds are specifically referred to.
The term derivative includes also the corresponding derivatives bearing a sugar residue.
When somatostatins bear a sugar residue, this is preferably coupled to a N-terminal amino group and/or to at least one amino group present in a peptide side chain, more preferably to a N-terminal amino group. Such compounds and their preparation are disclosed, e.g. in WO 88/02756.

The term octreotide derivatives includes those including the moiety 
        *                    *
-D-Phe-Cys-Phe-DTrp-Lys-Thr-Cys-
having a bridge between the Cys residues.

Particularly preferred derivatives are N^{α}-[α-glucosyl-(1-4-deoxyfructosyl]-DPhe-Cys-Phe-DTrp-Lys-Thr-Cys-Thr-ol and N^{α}-[β-deoxyfructosyl-DPhe-Cys-Phe-DTrp-Lys-Thr-Cys-Thr-ol, each having a bridge between the -Cys- moieties, preferably in acetate salt form and described in Examples 2 and 1 respectively of the above mentioned application.

The somatostatins may exist e.g. in free form, salt form or in the form of complexes thereof. Acid addition salts may be formed with e.g. organic acids, polymeric acids and inorganic acids. Acid addition salts include e.g. the hydrochloride and acetates. Complexes are e.g. formed from somatostatins on addition of inorganic substances, e.g inorganic salts or hydroxides such as Ca- and Zn-salts and/or an addition of polymeric organic substance.

The acetate salt is a preferred salt for such formulations. The pamoate salt is also useful.

The pamoate may be obtained in conventional manner, e.g. by reacting embonic acid (pamoic acid) with octreotide, e.g. in free base form. The octreotide pamoate is described in the UK Patent application GB 2,234,896 A, Example 10.

The reaction may be effected in a polar solvent, e.g. at room temperature.

The somatostatins are indicated for use in the treatment of disorders wherein long term application of the drug is envisaged, e.g. disorders with an aetiology comprising or associated with excess GH-secretion, e.g. in the treatment of acromegaly, for use in the treatment of gastrointestinal disorders, for example, in the treatment of peptic ulcers, enterocutaneous and pancreaticocutaneous fistula, irritable bowel syndrome, dumping syndrome, watery diarrhea syndrome, acute pancreatitis and gastroenterophathic endocrine tumors (e.g. vipomas, GRFomas, glucagonomas, insulinomas, gastrinomas and carcinoid tumors) as well as gastro-intestinal bleeding, breast cancer and complications concerned with diabetes. Further the somatostatins are indicated for the treatment of arthritis conditions, e.g. rheumatoid arthritis.

As a side effect of therapy in which somatostatins are administered sometimes, at least for octreotide, the formation of symptomatic gallstones has been observed. However, little has been published on the mechanism of formation of these gallstones or their compositions.

The cholanic acids of the formula I described before, as well as their pharmaceutically acceptable salts, are generally known per se. The compound having the 7α-hydroxyl group has been described in the Merck Index, Ninth Edition (1976), under monograph number 2010, in which it is denoted as chenodeoxycholic acid. The compound having the 7β-hydroxyl group is denoted as ursodeoxycholic acid and has been described under monograph number 9551.
Both cholanic acids are of natural origin and are, whether alone or, preferably, in combination, medically indicated in cholesterin gallstone dissolution treatment as described in Digestive Diseases and Sciences, Vol. 31, No. 10,1032-1040 (1986), Die Medizinische Welt, No. 45, 1489-1495 (1987), Gut, Vol. 28, No. 10, October 1987, A.1360. The cholanic acids are orally administered.

Based on data in "Drug Evaluations", 6th edition, American Medical Association, Chicago, Illinois the daily dosis for gallstone treatment is about at least 1000 mg/75 kg of body weight per day for chenodeoxycholic acid and 1200 - 1500 mg/75 kg of body weight per day for urso deoxycholic acid.

According to "The Pharmacological Basis of Therapeutics", Eighth edition, Goodman and Gilman, Pergamon Press, the dissolution of gallstones can also be performed by administering a combination of chenodeoxycholic acid and ursodeoxycholic acid at lower dosages, e.g. 5 mg/kg body weight/day = 375 mg/75 kg of body weight/day of each compound (page 931).

As generally known for peptides, the development of appropriate and effective somatostatin administration forms has caused many problems.

Being peptides, the somatostatins are easily decomposed by the indigestion juices after oral administration. Moreover, transport across the mucous membranes in the body, whether the stomach or intestines or in the mouth, the nose or the rectum is inefficient. For this reason the somatostatins are parenterally administered to humans. No other commercially available route of administration is available, despite the well known disadvantages of parenteral administration.

According to Gastroenterology Vol. 96, No. 5, Part 2, page A 580 (1989), Abstract of Papers, gallstones formed under the influence of a long term therapy with the specific somatostatin Sandostatin® (= Octreotide) were treated during further octreotide administration with ursodeoxy cholic acid resulting in the dissolution of the stones in just one patient. The somatostatin was given parenterally at a known dose of about 450 microgram, the ursodeoxycholic acid orally and thus no pharmaceutical composition was given in which somatostatins were combined with urso- and/or chenodeoxycholic acid.

We have now surprisingly discovered that both cholanic acids mentioned before, i.e. one of them or both in combination, can be used, e.g. at low dosages, for an improved resorption of a somatostatin through the mucous membrane e.g. of the gastrointestinal tract, as determined in the following experiment:

Wistar rats (about 300 g body weight) were fasted for 12 hours and anaesthetised with urethane (2 doses of 0.7 mg/kg i.p.). The abdomen was opened to gain access to the gastrointestinal tract in particular the jejunum.
A solution of the pharmaceutical composition according to the invention or of the somatostatin as a control or of a somatostatin in combination with sodium taurocholate, a resorption enhancer, known to be used in combination with e.g. salmon calcitonin or insulin for an improved resorption through the mucous membrane of the gastrointestinal tract, was injected into the gastrointestinal tract, e.g. the jejunum. Blood samples (e.g. 1 ml) are obtained 20 minutes, and 1 and 2 hours after administration from the vena cava. The blood is centrifuged and the somatostatin concentration in the plasma determined in conventional manner using a radio-immunoassay method.

The relative bioavailability (AUCₒ² = Area under the curve over 2 hours) is determined and expressed in the following table, in which the AUC is expressed as ng/ml.hour and Cₘₐₓ as ng/ml:

**TABLE A**

| Administered 0.5 ml of NaCl (0.9 % W/V) solution | AUCₒ² | | Cₘₐₓ | | Bioavail. | |
|---|---|---|---|---|---|---|
| containing further: | median | mean | sem | mean | sem | rel. % |
| 100 microg. of octreotide (reference) | 2.06 | 2.26 | 0.46 | 2.23 | 0.55 | 100 % |
| 100 microg. of octreotide + 5 mg of chenodeoxycholic acid | 80.78 | 84.26 | 21.29 | 63.76 | 18.48 | 3921.36 |
| 100 microg. of octreotide + 5 mg of ursodeoxycholic acid | 8.35 | 10.33 | 2.94 | 10.76 | 2.98 | 405.39 |
| 100 microg. of octreotide + 5 mg of sodium taurocholate | 2.91 | 5.40 | 2.22 | 4.02 | 1.47 | 141.26 |
| sem = standard error of the mean. | | | | | | |

The invention thus provides a pharmaceutical composition containing a somatostatin, its analgoues and derivatives, and at least one cholanic acid of the formula I, mentioned before, and having, when administered to the jejunum of a rat a relative somatostatin bioavailability of at least 400%, compared with the same composition without the resorption promoter.

Whereas the sodium taurocholate containing solution exhibits only a small octreotide bioavailability improvement in relation to the reference (improvement factor = 1.4), the solutions with ursodeoxycholic acid and especially chenodeoxycholic acid show considerably better improvement factors of about 4 and 39 respectively.

The invention thus provides the administration of the cholanic acids of formula I via the same transmucosal, preferably oral, route as the administration of a somatostatin.

The invention preferably provides pharmaceutical compositions containing a somatostatin, especially octreotide or its derivatives, chenodeoxycholic acid and/or ursodeoxycholic acid and/or their pharmaceutically acceptable salts.

In a further aspect the invention provides a process for the production of a pharmaceutical composition of the invention, which comprises working up the somatostatin and the resorption promoter.
The cholanic acid may be worked up in any pharmaceutically acceptable condition, e.g. as a precursor, or as a salt, e.g. an alkaline metal salt.

The pharmaceutical composition may be produced in conventional manner, using, if desired, appropriate excipients for the route of administration particularly contemplated, e.g. nasal, rectal or, preferably, oral.

Preferably the composition contains no water and is in the solid state. The invention relates particularly to pharmaceutical compositions for nasal, rectal and oral administration.
The compositions may be prepared by conventional techniques to be in conventional forms for transmucosal administration, for example, capsules, tablets, suppositories, dispersible powders. Preferably the cholanic acid of formula I is the sole bile salt present.

The exact dose of somatostatin to be used may be ascertained in conventional animal or clinical studies. For example, the dose may be ascertained by comparative bioavailability trials with other formulations with a known therapeutic effect, the dose being chosen to produce in the steady state comparable drug levels to therapeutically effective levels, e.g. on parenteral administration.

In the compositions of the invention the dosage of the cholanic acids can be chosen within wide ranges: at lower dosages the cholanic acids show the resorption promoting effect on somatostatins, at higher dosages, the cholanic acids - additionally - have a gallstone dissolving effect. In dosages between these extreme limits the cholanic acids surprisingly inhibit gallstone formation and thus have a gallstone prophylactic effect.

The compositions of the invention in which the amount of cholanic acid is chosen such that it has a resorption promoting and additionally a gallstone prophylactic effect are the most preferred.

The compositions of the invention open up the possibility of a more convenient method of administration of sandostatin compositions.

The compositions are preferably so formulated that the therapeutic daily dose of somatostatin and the daily dose of cholanic acid for a resorption promoting and preferably additionally a gallstone prophylactic effect are incorporated in the same dosage units. However, the invention also relates to preparations, in which the daily dosages of somatostatin and of cholanic acids are separate, but are administrable both for the same transmucosal route, whether rectal, nasal or oral.

Hence, the invention also provides a package containing unit dosages of a cholanic acid of formula I and of a somatostatin, as a combined preparation for simultaneous, separate or sequential use via the same transmucosal route, preferably the oral route, in a therapy for which the somatostatin is indicated.

Such unit dosages may be made in analogous manner to the pharmaceutical compositions of the invention.

Conveniently, the compositions are in unit dosage form or may be divided into or administered as unit dosages each preferably containing e.g. up to 35 mg of a somatostatin, the exact amount depending on e.g. the active ingredient, the type of illness to be treated and the mode of administration. When the used somatostatin is octreotide or vapreotide preferably 1 to 10 mg is used per unit dosage form.

The unit dosage form contains preferably 5 to 500, especially up to 250 mg of one of the cholanic acids or of a mixture of both for a resorption promoting effect on the somatostatin used. For an additional effective gallstone prophylaxis the unit dosage may contain such an amount of cholanic acid, that its daily dosis is up to 1500 mg, e.g. up to 1000 mg. If a combination of urso- and chenodeoxycholic acids is used, the dosage form may contain such amounts that their daily doses are e.g. up to 1000 mg/day, e.g. up to 400 mg/day of each component.

The invention is illustrated by the following pharmaceutical compositions:

### EXAMPLE 1:

| Capsules for oral application: | |
|---|---|
| Octreotide | 2,3 mg* (aequivalent to 2 mg somatostatin) |
| Chenodeoxycholic acid | 150 mg |
| Microcrystalline cellulose | 100 mg |
| Lactose | 50 mg |

| | |
|---|---|
| * the acetate salt | |

### EXAMPLE 2:

| Suppositoria for rectal application: | |
|---|---|
| Octreotide | 5,8 mg* (aequivalent to 5 mg somatostatin) |
| anhydrous citric acid | 0.78 |
| Trisodium citrate-hydrate | 0.50 |
| Mannitol | 48.651 |
| Chenodeoxycholic acid | 150.0 |
| Suppositorium base A* | 1300.0 |
| | 1̅5̅0̅0̅.̅0̅ m̅g̅ |

| | |
|---|---|
| * the acetate salt | |
| ** Witepsol ^{R} H.12, linear (C₁₀₋₁₈) fatty acid glyceride, Melting range 32-33.5°, solidification range 29-33°C Obtainable from Dynamit Nobel, Germany. | |

### EXAMPLE 3:

| Solutions for nasal application: | |
|---|---|
| Octreotide | 2,3 mg |
| Sodium citrate - dihydrate | 10,00 mg |
| Citric acid - monohydrate | 10,00 mg |
| Chenodeoxycholic acid | 25,00 mg |
| Ethylenediaminetetracetic acid disodium salt | 1,0 mg |
| Benzalkonium chloride | 0,2 mg |
| | 4̅8̅,̅5̅ m̅g̅ |

The powder is sprayed in a quantity of about 6 mg per nostril. When administered 4 times daily, the dose of octreotide is sufficient for therapeutic purposes.

Instead of octreotide other somatostatins can be formulated. Instead of chenodeoxycholic acid the same amount of ursodeoxycholic acid can be used.

## Claims

1. A pharmaceutical composition, containing a somatostatin and at least one cholanic acid of formula I 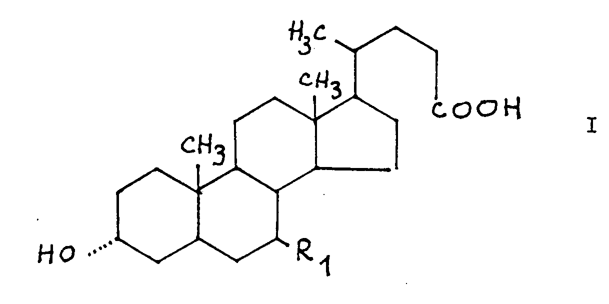 wherein R₁ = α- or β-OH. as a resorption promoter with a gallstone eliminating/preventing or size reducing activity, adapted for administration via a transmucosal route.

2. A composition according to claim 1, having, when administered to the jejunum of a rat, a relative somatostatin bioavailability of at least 400 %, compared with the same compositions without the resorption promoter.

3. A composition according to claim 2, in which the cholanic acid of formula I is ursodeoxycholic acid.

4. A composition according to claim 2, in which the cholanic acid of formula I is chenodeoxycholic acid.

5. A composition according to any one of claims 1 to 4, in which the somatostatin is octreotide.

6. A composition according to any one of claims 1 to 5 for nasal, rectal or oral administration.

7. A composition according to any one of claims 1 to 6, containing up to 35 mg of a somatostatin per dosage unit.

8. A composition according to any one of claims 1 to 7 containing from 1 to 10 mg of octreotide per dosage unit.

9. A composition according to any one of claims 1 to 8 containing from 5 to 500 mg of the cholanic acid compound per dosage unit.

10. A composition according to any one of claims 1 to 9 in solid state.

11. A process for the production of a pharmaceutical composition of any one of claims 1 to 10 which comprises working up the somatostatin and the resorption promoter.

12. A package containing unit dosages of a cholanic acid of formula I given in claim 1 and of a somatostatin, as a combined preparation for simultaneous, separate or sequential use via the same transmucosal route in a therapy for which the somatostatin is indicated.

13. A package according to claims 12, for oral application of the unit dosages.

## Patentansprüche

1. Pharmazeutische Komposition die ein Somatostatin und als Resorptionsförderer mit gallensteineliminierender, - verhindernder oder-verkleinernder Wirkung mindestens eine Cholansäure der Formel I 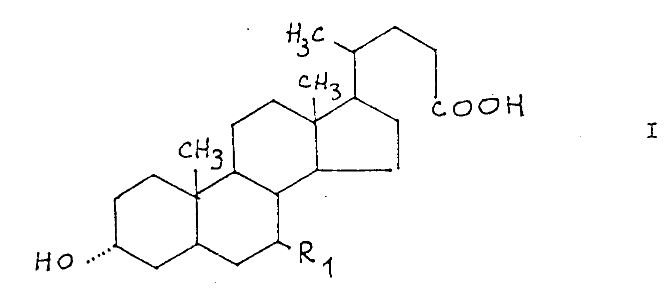 enthält
worin R₁= α - oder β - OH, zur Applikation über den transmukosalen Weg ausgestaltet.

2. Komposition gemäss Anspruch 1 welche eine relative Somatostatinbioverfügbarkeit von mindestens 400% gegenüber der gleichen Komposition ohne Resorptionsförderer aufweist, wenn sie ins Jejunum einer Ratte gebracht wird.

3. Komposition gemäss Anspruch 2, mit Ursodeoxycholsäure als Cholansäure der Formel 1.

4. Komposition gemäss Anspruch 2, mit Chenodeoxycholsäure als Cholansäure der Formel 1.

5. Komposition gemäss einem der Ansprüche 1 bis 4 mit Octreotid als Somatostatin.

6. Komposition gemäss einem der Ansprüche 1 bis 5 zur nasalen, rektalen oder oralen Applikation.

7. Komposition gemäss einem der Ansprüche 1 bis 6 mit bis 35 mg eines Somatostatins per Dosierungseinheit.

8. Komposition gemäss einem der Ansprüche 1 bis 7 mit 1 bis 10 mg Octreotid pro Dosierungseinheit.

9. Komposition gemäss einem der Ansprüche 1 bis 8 mit 5 bis 500 mg der Cholansäureverbindung pro Dosierungseinheit.

10. Komposition gemäss einem der Ansprüche 1 bis 9 in festem Zustand.

11. Verfahren zur Herstellung einer pharmazeutischen Komposition gemäss einem der Ansprüche 1 bis 10, dadurch gekennzeichnet, dass man darin das Somatostatin und den Resorptionsförderer verarbeitet.

12. Verpackung mit Dosierungseinheiten einer Cholansäure der Formel 1 und eines Somatostatins als Kombinationspräparat für den gleichzeitigen separaten oder nachfolgenden Einsatz über den gleichen transmukosalen Applikationsweg in einer Therapie für welche das Somatostatin vorgesehen ist.

13. Verpackung gemäss Anspruch 12, zur oralen Applikation der Dosierungseinheiten.

## Revendications

1. Une composition pharmaceutique, contenant une somatostatine et au moins un acide cholanique de formule I 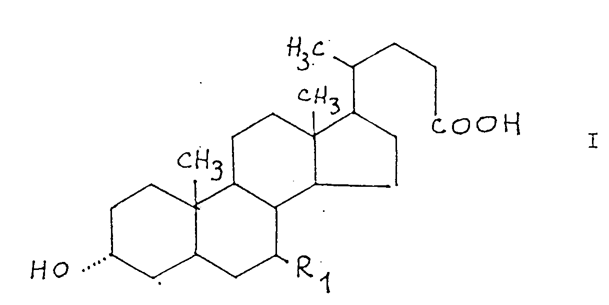 dans laquelle R₁ = α- or β-OH, comme promoteur de résorption ayant une activité permettant d'éliminer les calculs biliaires, d'empêcher la formation des calculs biliaires ou de réduire la dimension des calculs biliaires, et adaptée pour l'administration à travers les muqueuses.

2. Une composition selon la revendication 1 ayant, après administration au jejunum d'un rat, une biodisponibilité relative de somatostatine d'au moins 400%, par rapport à la même composition exempte de promoteur de résorption.

3. Une composition selon la revendication 2, dans laquelle l'acide cholanique de formule I est l' acide ursodésoxycholique.

4. Une composition selon la revendication 2, dans laquelle l' acide cholanique de formule I est l'acide chénodésoxycholique.

5. Une composition selon l'une quelconque des revendications 1 à 4, dans laquelle la somatostatine est l'octréotide.

6. Une composition selon l'une quelconque des revendications 1 à 5, pour l'administration nasale, rectale ou orale.

7. Une composition selon l'une quelconque des revendications 1 à 6, contenant jusqu'à 35 mg de somatostatine par dose unitaire.

8. Une composition selon l'une quelconque des revendications 1 à 7, contenant de 1 à 10 mg d'octréotide par dose unitaire.

9. Une composition selon l'une quelconque des revendications 1 à 8, contenant de 5 à 500 mg d'acide cholanique par dose unitaire.

10. Une composition selon l'une quelconque des revendications 1 à 9, sous forme solide.

11. Un procédé de préparation d'une composition pharmaceutique selon l'une quelconque des revendications 1 à 10, qui comprend le traitement de la somatostatine et du promoteur de résorption.

12. Un emballage contenant des doses unitaires d'un acide cholanique de formule I spécifié à la revendication 1 et d'une somatostatine, comme préparation combinée pour l'administration simultanée, séparée ou séquentielle à travers les muqueuses dans une thérapie pour laquelle la somatostatine est indiquée.

13. Un emballage selon la revendication 12, pour l'administration par voie orale de doses unitaires.
